# EUROPEAN PATENT APPLICATION

(11) **EP 1 690 808 A1**
(43) Date of publication of application: **16.08.2006**
(21) Application number: 04819304.9
(22) Date of filing: 15.11.2004
(51) Int. Cl.: B65D 83/14, B05B 9/04

(54) **SUBDIVISIONAL FIXED AMOUNT DISTRIBUTING DEVICE FOR AEROSOL CONTAINERS**

(30) Priority: 25.11.2003 JP 2003394337
(71) Applicant: TAISHO PHARMACEUTICAL CO., LTD, Tokyo 170-8633 (JP); Toyo Aerosol Industry Co., Ltd., Tokyo 100-0011 (JP)
(72) Inventor: MATSUMOTO, Keiko, Taisho Pharmaceutical Co., Ltd., Toshima-ku, Tokyo 1708633 (JP); OGATA, Ken, Irumagun, Saitama 3500438 (JP)
(74) Representative: Kloiber, Thomas
(86) International application number: PCT/JP2004/016951
(87) International publication number: WO 2005/051803

(57) **Abstract**

The frequency which allows continuous injection of a fixed amount in the use of one cycle is controlled so as to prevent the use of aerosol contents in excess of a control amount. Further, it is made possible to inject all amount to the outside each time by a fixed-amount injection valve, thereby simplifying the mechanism and preventing damage to the distributing device due to the influence of outside air temperature. A lower receiving blade 31 is disposed in an outer sleeve 14 fixed to the upper end of an aerosol container 1 through vertical insertion intervals 32, 27 and is inserted in the lower insertion interval 32 by positional shift, thereby a fitting piece 22 which allows pressing by a stem 7 is projectingly formed on a pushing body. Further, an upper receiving blade 36 which allows the pushing body 15 to move in the same direction as the direction of said positional shift is formed in the inner surface of an upper sleeve 24 through the upper insertion interval 37. After the positional shifting of the pushing body 15 accompanying a plurality of times of pressing, movement into the lower insertion interval 32 is made impossible, thereby making fixed-amount injection of aerosol contents impossible.

## Description

### Technical Field

This invention relates to a subdivided fixed amount distributing apparatus for aerosol containers allowing only use of a fixed amount of aerosol contents such as medical products, hair care products, and cosmetic products in which a use amount for one cycle, such as e.g., one day, several days, or one week, is restricted.

### Background Technology

Some aerosol contents contained in an aerosol container, such as medical products, hair care products, and cosmetic products may be used directly for human bodies. If such aerosol contents are used exceedingly in an amount more than a prescribed use amount, however, such use may cause harmful effects on human beings, or may result in wasteful use. As disclosed in Japanese Unexamined Patent Publication No.2001-232249, a subdivided amount distributing apparatus for aerosol containers has been developed in which the apparatus capable of spraying a prescribed fixed amount usable in a certain period multiple times is connected to a stem of an aerosol container and disposed at an exterior of the aerosol container.

This subdivided amount distributing apparatus for aerosol containers is formed with a fixed amount chamber and a fixed amount injection valve, respectively, in the apparatus, and the aerosol contents of a fixed amount are introduced from the aerosol container main body into the fixed amount chamber and stored therein. The interior of the fixed amount chamber is normally pressurized with a piston, and the aerosol contents contained in the fixed amount chamber are injected multiple times in a subdivided manner through the fixed amount injection valve. Fixed amount injection during a prescribed period is allowed by injection of a fixed amount one or more times, thereby bringing advantages realizing distribution of aerosol contents suitable for medical products, hair products, and cosmetic products, which are otherwise not favorable when used in an amount of a prescribed amount or more or used in a wasteful manner.

### Summary of the Invention

### Problems to be solved by the Invention

The prior publicly known invention includes the subdivided amount distributing apparatus is disposed at an exterior of the aerosol container. With such a subdivided fixed amount distributing apparatus connected to the exterior of the aerosol container, however, the prescribed amounts in proportion to one day or one week are stored in the fixed amount chamber in the prescribed period in a state that the aerosol contents are pressurized, so that the volume of the aerosol contents may be expanded due to changes of the outer temperature in some occasions, and so that the distributing apparatus disposed at the exterior of the aerosol container may be damaged. Because the contents are injected while the fixed amount chamber is pressurized by the piston, injection is made continuously upon gasification of liquid gas remaining at a space between the piston and the fixed amount chamber even after the injection of the liquid portion of the aerosol contents is completed, thereby having a defect to inject only gasified gas. In addition, the fixed amount chamber and the fixed amount injection valve, both, are required to be built in the distributing apparatus, thereby rendering assembly of the apparatus complicated and manufacturing costs high.

This invention, to solve the above problems, does not require to have a large fixed amount chamber able to inject the contents plural times at an exterior of the aerosol container, because the aerosol contents of a fixed amount contained in the aerosol container main body are injected after filled in the fixed amount chamber provided in the aerosol container, so that the distributing apparatus is prevented from receiving damages due to changes of the external temperature, thereby allowing the aerosol container to be used stably for a long period of time by safe use of the aerosol container, and thereby reducing the manufacturing costs.

### Means to be solved by the Invention

This invention has a feature in which a subdivided fixed amount distributing apparatus for aerosol container includes: an outer sleeve secured to a top of the aerosol container and formed with a penetration opening at a center thereof; a nozzle body disposed in the penetration opening of the outer sleeve and formed with a nozzle communicating with a stem; a pushing body penetrated by the nozzle of the nozzle body at a center thereof and urged in an upper direction by a coil spring wound around the nozzle body, the pushing body pushing the stem according to pushing down operation to open a fixed amount injection valve disposed in the aerosol container thereby allowing injection of entire amounts of aerosol contents within the fixed amount injection valve, the pushing body being pivotally movable with respect to the nozzle body and the outer sleeve; and an upper sleeve attaching the pushing body slidably in an up and down direction at a center opening thereof, the upper sleeve being secured to the outer sleeve at a lower end thereof, wherein plural lower receiving blades whose top end forms a portion tapered at one corner are arranged annularly at an outer periphery of the penetration opening of the outer sleeve via lower insertion intervals extending in an up and down direction, wherein a flat portion having the same level as the lower receiving blades is formed at an end of the lower receiving blades in this arrangement direction, wherein a fitting piece is formed in projecting from a lower surface of the pushing body for allowing the pushing body to be pushed to the stem by pushing the pushing body to the lower receiving blades from an upper surface of the blades along the portion tapered at one corner as to be moved and inserted into the lower insertion interval, wherein plural upper receiving blades for moving the pushing body in the same direction as the moving direction of the pushing body by hitting a top of the fitting piece according to the pushing body's lifting up due to release of pushing operation to the pushing body are arranged annularly at an inner surface of the upper sleeve in having a lower surface tapered at one corner via upper insertion intervals, and wherein the fixed amount injection of the aerosol contents is disabled by pushing operation plural times to the pushing body and by disabling movement of the pushing body into the lower insertion interval upon rendering the fitting piece hit the flat portion after the pushing body is moved according to the pushing operation.

The pushing body may be formed with a pushing projection at an upper surface thereof to be in pressurized contact with a user. The pushing projection of the pushing body may be formed in coupling with the fitting piece. The pushing body may be formed with the pushing projection and the fitting piece, which are formed separately.

### Advantages of the Invention

This invention simplifies the mechanism by injecting the whole amount each time to the exterior with the fixed amount injection valve contained in the aerosol container main body and prevents the distributing apparatus from receiving damages due to influences of external temperature. This invention, with the simple safe mechanism, has an advantage to render the aerosol container usable safely for a long time and to render the manufacturing costs inexpensive by limiting number of times in series of injections for fixed amount at one cycle and by preventing the aerosol contents from being used in an amount more than the prescribed amount, where medical products, hair care products, cosmetic products, etc. which are not suitable for use of a prescribed amount or more, are used as aerosol contents.

### Brief Description of the Drawings

Fig. 1 is an exploded perspective view showing the first embodiment of the invention;
Fig. 2 is a cross section showing the first embodiment of the invention in an assembled state;
Fig. 3 is a cross section showing a fixed amount injection state;
Fig. 4 is a cross section showing a state that a fitting piece hits a flat portion after completion of fixed amount injection for one cycle;
Fig. 5 is an exploded view showing a state that the fitting piece is disposed at an upper flat portion;
Fig. 6 is an exploded view showing a state that the fitting piece fits to a lower insertion interval;
Fig. 7 is an exploded view showing a state that the fitting piece hits the flat portion;

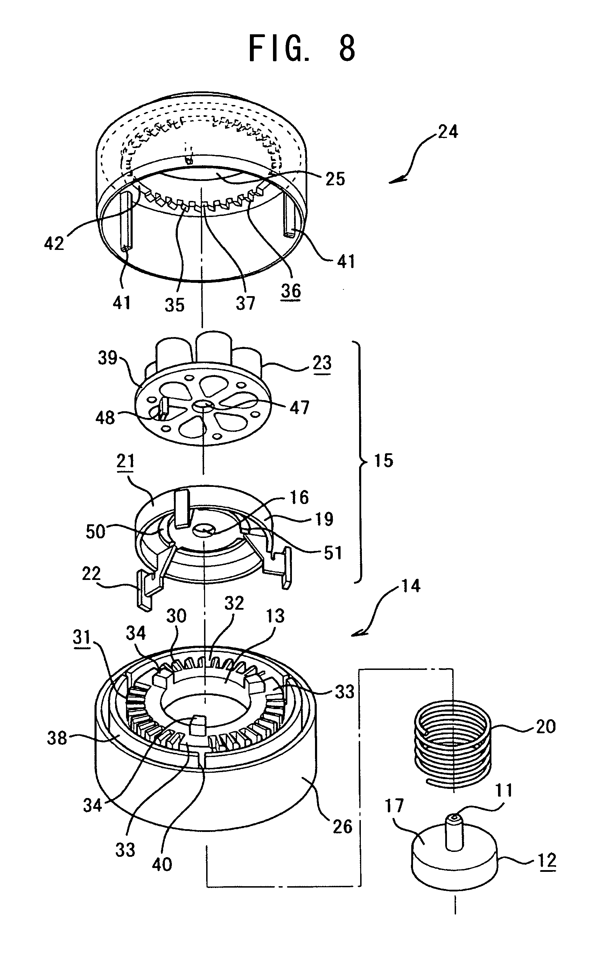

Fig. 8 is an exploded view showing the second embodiment of the invention;
Fig. 9 is an exploded view showing a state that the fitting piece fits into an upper insertion interval in the second embodiment;
Fig. 10 is an exploded view showing a state that the fitting piece hits the flat portion in the second embodiment; and
Fig. 11 is a perspective view showing a pushing projection portion in the second embodiment.

As description of reference numbers: 1 aerosol container, 2 fixed amount injection valve, 7 stem, 11 nozzle, 12 nozzle body, 13 penetration opening, 14 outer sleeve, 15 pushing body; 20 coil spring, 22 fitting piece, 23 pushing projection, 24 upper sleeve, 25 center opening, 30 upper end surface, 31 lower receiving blade, 32 lower insertion interval, 33 flat portion, 35 lower end surface, 36 upper receiving blade, 37 upper insertion interval.

### Description of the Preferred Embodiments

### [First Embodiment]

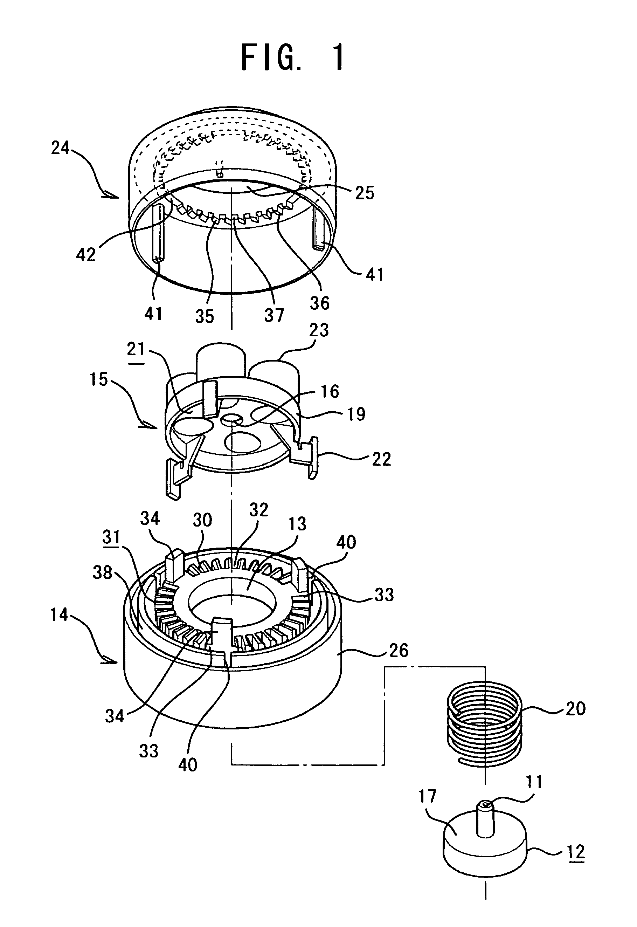

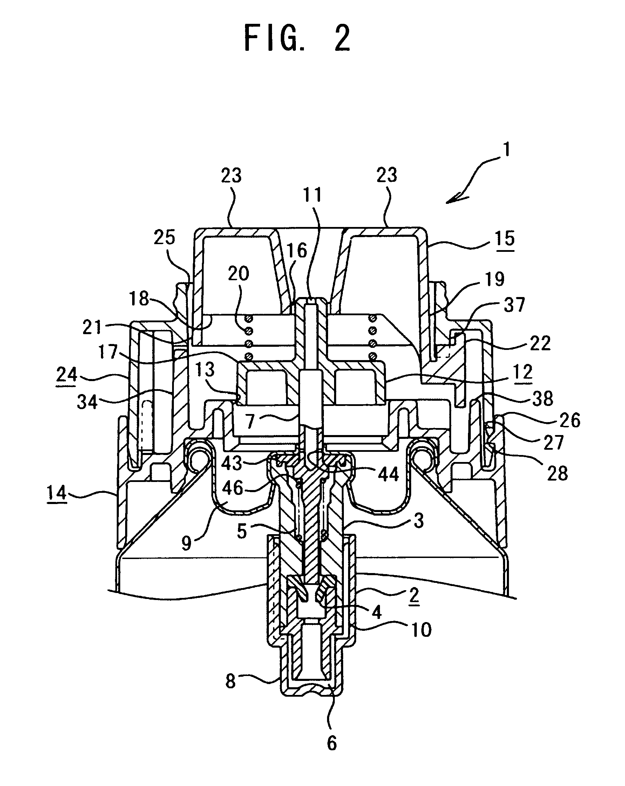

Referring to Fig. 1 through Fig. 7, a first embodiment using a hair growth agent injected plural times during one cycle use but subjecting to limitation of the whole use amount of injections during the plural times, is described hereinafter. Numeral 1 is an aerosol container, and as shown in Fig. 2 to Fig. 4, a fixed amount injection valve 2 whose top end is secured to a lid 9 is disposed in the aerosol container 1. A stem 7 disposed in the fixed amount injection valve 2 is formed penetrating a stem gasket 43 provided at an inner surface of the lid 9, and an orifice 44 is arranged on an outer side of the stem gasket 43 during a non-pushing state whereas the orifice 44 is arranged as inserted in a fixed amount chamber 5 during a pushing state.

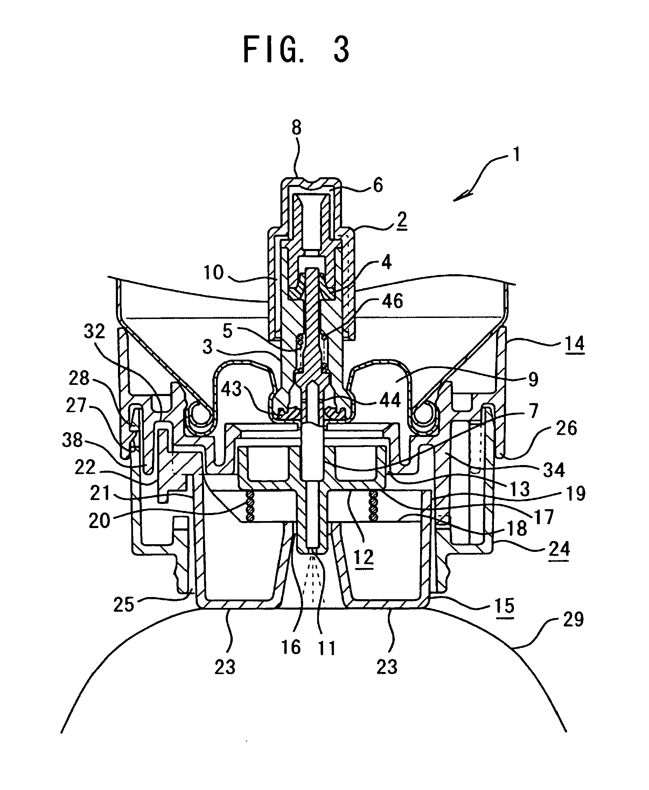

The fixed amount injection valve 2 is formed with the fixed amount chamber 5 and a content introduction chamber 6 via an annular partition gasket 4 in a housing 3. The partition gasket 4 comes close contact to the outer periphery of the stem 7 as shown in Fig. 3 to disconnect the fixed amount chamber 5 from the content introduction chamber 6, and the orifice 44 of the stem 7 is introduced into the interior of the fixed amount chamber 5 where the stem 7 is pushed and penetrated through the partition gasket 4 during use in a manner of up side down, so that the aerosol contents contained in the fixed amount chamber 5 only is discharged in the whole amount to the exterior. Upon release of pushing operation to the stem 7, as shown in Fig. 2, the stem 7 is returned to the original position to separate the partition gasket 4, thereby communicating the fixed amount chamber 5 with the content introduction chamber 6, and thereby introducing the aerosol contents contained in the content introduction chamber 6 into the fixed amount chamber 5.

The content introduction chamber 6 is normally in communication with an interior of the aerosol container 1 through an introduction route 10 provided between the outer periphery of the housing 3 and an outer sleeve 8 of the housing 3. It is to be noted that in this embodiment the fixed amount injection valve 2 thus formed is used but the structure is not limited as far as a fixed amount valve 2 used in other embodiments can inject the aerosol contents of a prescribed amount to the exterior by pushing operation of the stem 7.

As shown in Fig. 2, a nozzle body 12 forming a cylindrical shape nozzle 11 is formed as connecting to an upper end of the step 7, thereby communicating the stem 7 and the nozzle 11. An outer sleeve 14 opening a penetration opening 13 at a center thereof is secured to an upper end of the aerosol container 1 so that the nozzle body 12 and the stem 7 penetrate the penetration opening13. This nozzle body 12 is disposed so as to be movable in an up and down direction in the penetration opening.

The nozzle 11 is inserted from an insertion opening 16 formed at a center of a substrate 21 of the pushing body 15, thereby arranging the pushing body 15 as to face to the penetration opening 13 of the outer sleeve 14. A coil spring 20 is provided, as wound, between a shoulder portion 17 of the nozzle body 12 and an engagement step 18 formed inside the pushing body 15 to urge the pushing body toward an external direction. The pushing body 15 is, as shown in Fig. 1, formed with the insertion opening 16 at the center of the substrate 21, with four pushing projections 23 as a united body on an outer peripheral surface of the insertion opening 16, and with an annular wall 19 formed in a projecting manner on a lower surface of the outer periphery thereof. Fitting pieces 22 are formed on a lower surface of the substrate 21 of the pushing body 15 as projecting toward an axial direction of the lower surface.

The pushing projections 23 of the pushing body 15 are inserted into a center opening 25 formed at an upper surface of an upper sleeve 24, and as shown in Fig. 2, the upper sleeve 24 is secured to the outer sleeve 14 by engaging engagement receiving portions 28 formed at a lower portion on the outer peripheral surface of the upper sleeve 24 with engagement projections 27 formed on an inner side of an outer side wall 26 of the outer sleeve 14. The pushing body 15 is isolated from the upper sleeve 24 in the center opening 25 of the upper sleeve 24 thus secured, and is arranged so as to be movable in the up and down direction as urged by the coil spring 20.

As shown in Fig. 1, lower receiving blades 31 whose upper end 30 has a tapered portion on one side is formed in a projecting manner on an outer periphery of the penetration opening 13 formed in the outer sleeve 14. The lower receiving blades 31 are arranged annularly via lower insertion intervals 32, and are placed so that a lower end of the fitting pieces 22 formed as projecting at the pushing body 15 can be inserted into the lower insertion intervals 32. Where the outer periphery of the penetration opening 13 is divided into three parts, the lower receiving blades 31 are continuously formed by ten pieces at each part with the lower insertion intervals 32, and flat portions 33 having the same level as the lower receiving blade 31 are formed at each end of one section at which no lower receiving blade 31 is continuously formed. Outer sleeve projections 34 formed in a projecting manner in a vertical direction are formed in continuation with the flat portions 33.

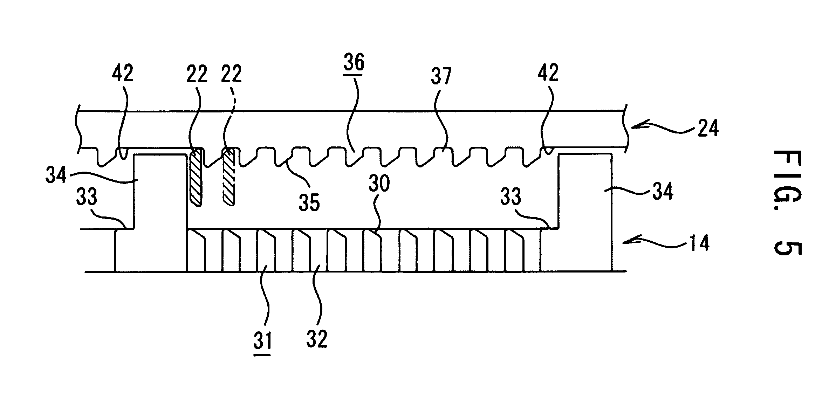

As shown in Fig. 5, upper receiving blades 36 whose lower end surface 35 is a tapered portion on one side in the opposite direction to the direction of the lower receiving blades 32 are formed in a projecting manner in the lower end direction at a lower end of the outer periphery of the center opening 25 of the upper sleeve 24. The upper receiving blades 36 are arranged annularly via upper insertion intervals 37 to which the fitting pieces 22 of the pushing body 15 can fit, and are continuously placed by ten pieces, upon divided into three parts, at each part at a lower end of the outer periphery of the center opening 25. Upper flat portions 42 having the same level of the upper end of the upper insertion intervals 37 are formed at each end of one section of the upper receiving blades 36.

With the outer sleeve 14 and the upper sleeve 24 thus structured, as shown in Fig. 1, engagement recess grooves 40 formed as recesses extending in the axial direction in the inner side wall 38 of the outer sleeve 14 are formed at three locations with equal intervals, and engagement projections 41 formed as protruded in extending in the axial direction of the inner side surface of the upper sleeve 24 are arranged at locations corresponding to the engagement recess grooves 40. The lower receiving blades 31 and the upper receiving blades 36 are positioned to right locations by engaging the engagement recess grooves 40 and the engagement projections 41 with one another. This positioning is done by engaging the engagement projections 41 with the engagement recess grooves 40 to connected the upper sleeve 24 to the outer sleeve 14 in a not pivotal manner, as shown in Figs. 5 to 7 to dispose the upper insertion intervals 37 of the upper sleeve 24 as corresponding to the lower receiving blades 31 of the outer sleeve 14, to dispose the lower insertion intervals 32 of the outer sleeve 14 as corresponding to the upper receiving blades 36, and to dispose the upper flat portions 42 to positions corresponding to the outer sleeve projections 34.

With the structure thus described, to perform subdivided fixed amount distributing injection of the aerosol contents, the pushing body 15 is moved pivotally in advance before the pushing body 15 is started to be pushed, and the fitting piece 22 of the pushing body 15 is arranged as shown in Fig. 5 between the lower receiving blade 31 and the upper flat portion 42 adjacent to the outer sleeve projection 34. Because the pushing body 15 is in a non-pushing state at that time, the fixed amount injection valve 2 disposed inside the aerosol container 1 is in a state that the fixed amount chamber 5 remains containing the aerosol contents.

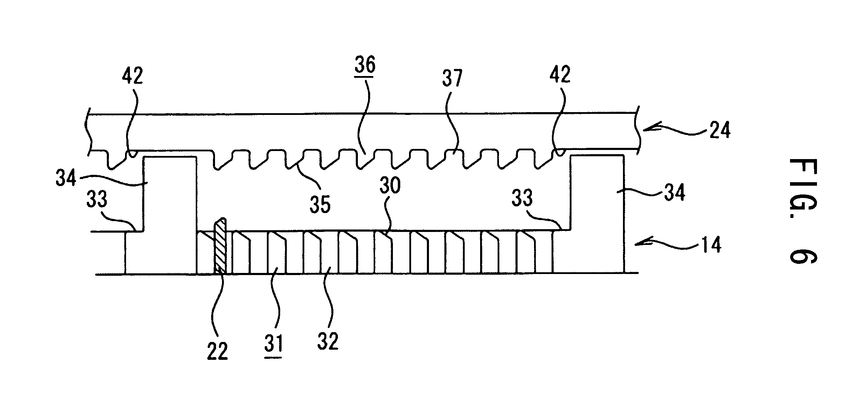

The aerosol container 1 is made up side down, and as shown in Fig. 3, the pushing body 15 is pushed in a bottom direction of the aerosol container 1 in opposing to urging force of the coil spring 20 while rendering the pushing projection 23 formed at the pushing body 15 in contact to an injection target portion 29 such as a head of human being. This pushing operation of the pushing projection 23 gives massage effects to the injection target portion 29. This pushing operation renders the fitting piece 22 move toward the outer sleeve 14 and hit the upper end surface 30 of the lower receiving blade 31, renders the fitting piece 22 slide into the lower insertion interval 32 along the portion tapered at one side formed at the upper end surface 30 and fit to the lower insertion interval 32 as shown in Fig. 6.

With this pushing operation at the pushing body 15, as shown in Fig. 3, the nozzle body 7 and the stem 7 move toward the bottom of the aerosol container 1. According to this movement, the orifice 44 disposed in the external direction of the stem gasket 43 at a time of non-pushing state is disposed inside the fixed amount chamber 5, and contacts to the aerosol contents in the fixed amount chamber 5. At the same time, the stem 7 penetrates the partition gasket 4 and cuts off communication between the content introduction chamber 6 and the fixed amount chamber 5. Therefore, the whole amount of the aerosol contents contained in the fixed amount chamber 5 is injected out of the nozzle 11 of the nozzle body 12 through the orifice 44 and the introduction opening of the stem 7.

When the pushing operation is stopped at a time that the injection is completed, the pushing body 15 moves toward the upper sleeve 24 by urging force of the coil spring 20 as shown in Fig. 2, and the nozzle body 12 and the stem 7 moved on the side of the bottom of the aerosol container 1 are backed to the original positions by the urging force of a stem spring 46. The fitting piece 22 disposed to the lower insertion interval 32 due to contact to the lower receiving blade 31 at a time of the pushing operation is moved toward the upper sleeve 24, and hits the upper receiving blade 36 located next to the upper flat portion 42 positioned before the pushing operation. The fitting piece 22 slides into the next upper insertion interval 37 along the portion tapered on one side formed at the lower end surface 35 of the upper receiving blade 36, and fits to the upper insertion interval 37 as shown with a single dot chain line in Fig. 5. With the steps described above, the pushing operation of the pushing body 15 of the first time and release of the pushing operation are completed.

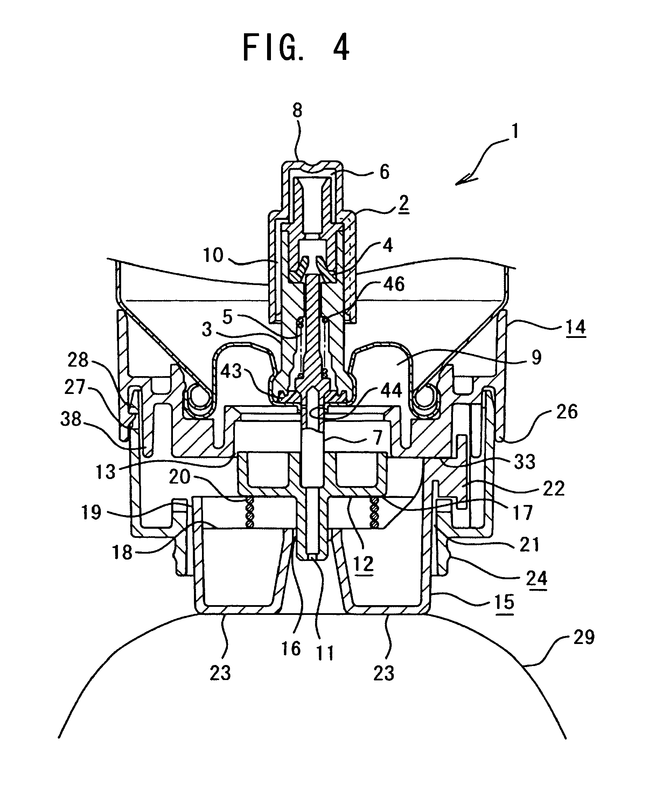

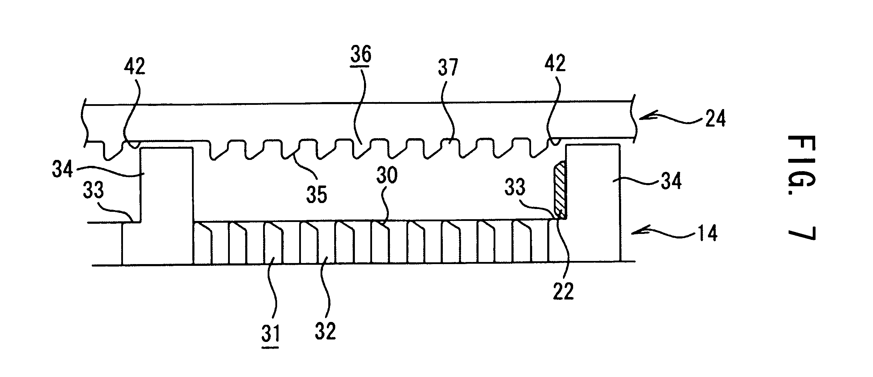

Thus, repeating pushing and stop of pushing of the pushing body 15, the fitting piece 22 moves sequentially between the upper insertion interval 37 and the lower insertion interval 32, and if the pushing body 15 is further pushed after the reciprocal movement is repeated ten times, the fitting piece 22 hits the flat portion 33 formed at the outer sleeve 14 as shown in Fig. 4 and Fig. 7. Therefore, the pushing body 15 moves toward the bottom of the aerosol container 1 in opposing to the urging force of the coil spring 20, but as shown in Fig. 4, the nozzle body 12 and the stem 7 cannot be moved toward the bottom of the aerosol because the fitting piece 22 remains on the flat portion 33. While the fitting piece 22 hits the flat portion 33, the fixed amount valve 2 is not made open, so that the aerosol contents cannot be injected. The use of the aerosol container 1 for one cycle is completed at that time, and no further injection of the aerosol contents is made subsequently under this situation, so that this apparatus can prevent the aerosol content from being used more than the prescribed amount.

Where use of one cycle is completed as described above, no following use is made subsequently, and as shown in Fig. 7, the pushing body 15 cannot be moved pivotally in the proceeding direction because the fitting piece 22 hits the outer sleeve projection 34 formed on the flat portion 33. Accordingly, to start the next use, the pushing body 15 is made in a non-pushing state, and moved pivotally in a direction opposite to the proceeding direction by manual handling, thereby repositioning, as shown in Fig. 5, the fitting piece 22 between the outer sleeve projection 34 and the upper flat portion 42 at which the fitting piece 22 is originally provided. This handling allows start of the use of the next cycle.

As described above, by pushing the pushing body 15 continuously after the fitting piece 22 is placed between the lower receiving blade 31 and the upper flat portion 42 adjacent to the outer sleeve projection 34 at the beginning, the fixed amount injections of the aerosol contents can be made ten times in total. The injection amount of the aerosol contents usable in a prescribed period can be limited to a prescribed amount, so that risks that aerosol contents are otherwise used more than the prescribed amount usable in one cycle can be avoided. For example, where the head of human being is the injection target portion 29, the aerosol contents of the total injection amount portion of one cycle can reach the whole head of human being entirely by injections ten times with changing the injection direction at each injection.

To restart the next cycle, it is required to take a special procedure in which the pushing body 15 is moved pivotally in the reverse direction to the proceeding direction of the previous cycle, and risks of use of an excessive use amount can be avoided because the user compulsively recognizes that it is important to use the contents within the prescribed amount by this procedure.

### [Second Embodiment]

In the above first embodiment, as shown in Fig. 1, the pushing projection 23 and the fitting piece 22 are formed as coupled by forming securely the pushing projections 23 on the upper surface of the substrate 21 formed at the pushing body 15 as a united body, but in the second embodiment, the pushing projection 23 and the fitting piece 22 are formed as separate members. The pushing projection 23 is formed with a center opening 47 at a center of a disc plate 39 as a separate body from the substrate 21, and with six cylindrical projections projecting on the upper surface. An engagement piece 48 is formed as projecting in an axial direction of the lower surface on the bottom surface of the pushing projection 23, and three engagement openings 50 are arranged annularly for inserting the engagement piece 48 at the periphery of the insertion opening 16 formed at a center of the substrate 21. Three fitting pieces 22 are arranged as projecting in the axial direction of the lower surface with equal intervals on the lower surface of the substrate 21 in substantially the same way as in the first embodiment. The substrate 21 and the pushing projection 23 are assembled to the aerosol container 1 upon engagement of the engagement opening 50 and the engagement piece 48.

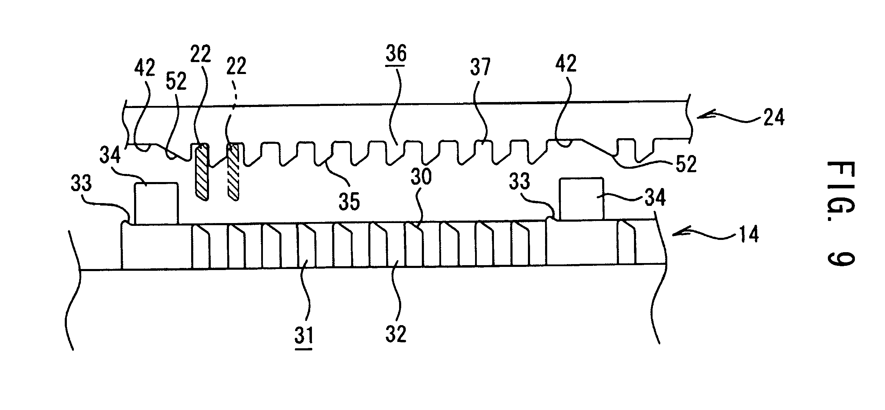

With the structure thus constituted, because the engagement piece 48 is movable freely in a range of the formed length of the engagement opening 50, the pushing projection 23 can move pivotally independently from the substrate 21 in the prescribed range in this embodiment. If the pushing body 15 is pushed, as shown in Fig. 9, the fitting piece 22 positioned at the upper insertion interval 37 moves along the tapered portion formed at the upper end surface 30 of the lower receiving blade 31 and fits to the lower insertion interval 32, but the pushing projection 23 does not necessarily move pivotally according to the movement of the fitting piece 22 because the pushing projection 23 is isolated from the substrate 21.

In a case where the pushing projection 23 performs massages to the injection target portion 29 during pushing injection operation, an occasion that the fitting piece 22 moves over the plural insertion intervals 32 may not happen as far as it is in the range of the formed length of the engagement opening 50 even where the pushing projection 23 moves to some extent. Because the substrate 21 is formed as separating form the pushing projection 23, unfavorable occasions such that the fitting piece 22 moves to the next fitting position according to pivotal movements of the pushing projection 23 can be avoided, even where the pushing projection 23 only is inadvertently moved pivotally in the range of the formed length of the engagement opening 50 in a state that injection is not performed. Therefore, the times of injections predetermined per one cycle use cannot be changed even where the pushing projection 23 moves pivotally to some extent, so that the whole injection amount of the aerosol contents to be injected at one cycle use can be maintained at a constant amount.

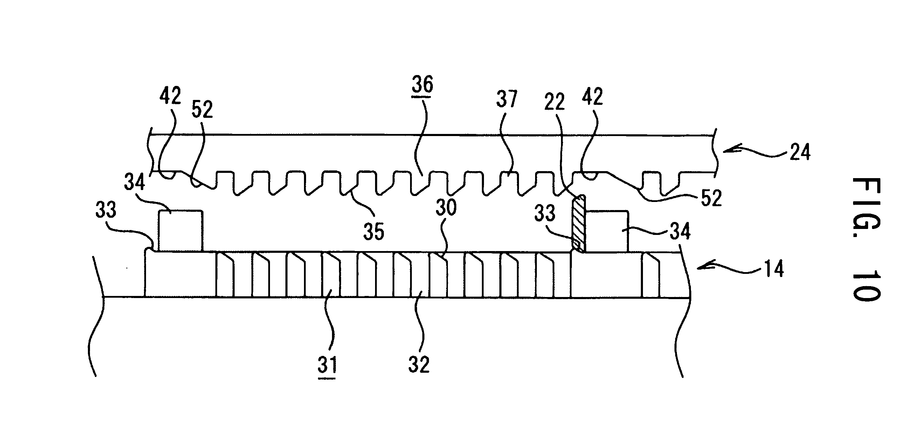

In this embodiment, in substantially the same way as in the first embodiment, the pushing operation of the pushing body 15 is disabled by repeating pushing and stop of pushing ten times continuously to render the fitting piece 22 reach the flat portion 33 as shown in Fig. 10. Use of one cycle is therefore completed at that time. To start use of the subsequent cycle, because the fitting piece 22 is required to be positioned at the subsequent fitting position, the pushing body 15 is made in a non-pushing state, and the pushing projection 23 is moved pivotally in the same direction as the proceeding direction of the fitting piece 22 of the pushing projection 23 by manual handling.

The engagement piece 48 formed at the pushing projection 23 hits an inner peripheral end surface 51 of the engagement opening 50 formed at the substrate 21, so that according to the pivotal movement of the pushing projection 23 the substrate 21 also pivotally moves in the same direction. With this pivotal movement, the fitting piece 22 rides over the outer sleeve projection 34 and ride over an inclined projection 52 along the tapered portion formed at a lower end of the center opening 25 of the upper sleeve 24, and as shown in Fig. 9, fits to the upper insertion interval 37 serving as the subsequent fitting position. With the above steps, the user can start use of the next cycle.

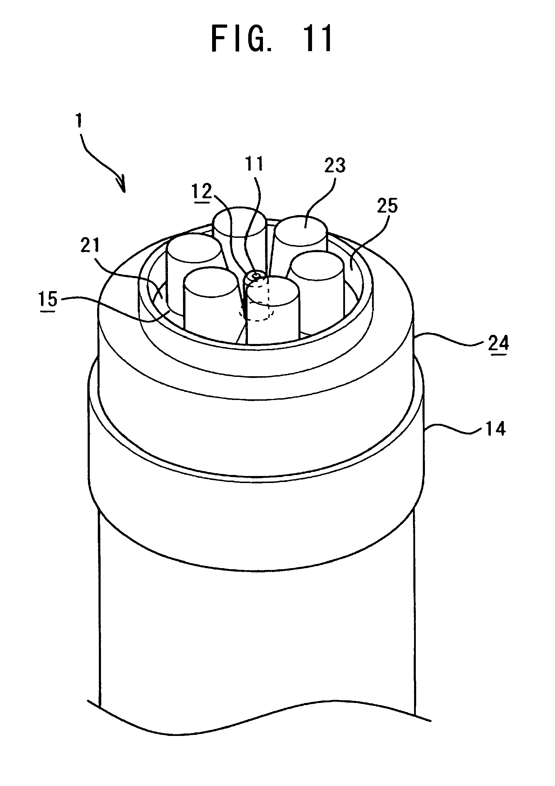

It is to be noted that to give stimulations to the head skin during use, four projections in the first embodiment, and six projections in the second embodiment as shown in Fig. 11 are formed, but in other embodiments, the pushing projection 23 can be formed in other shapes, and the number of projections cannot be limited to a certain number.

## Claims

1. A subdivided fixed amount distributing apparatus for aerosol container comprising:
an outer sleeve secured to a top of the aerosol container and formed with a penetration opening at a center thereof;
a nozzle body disposed in the penetration opening of the outer sleeve and formed with a nozzle communicating with a stem;
a pushing body penetrated by the nozzle of the nozzle body at a center thereof and urged in an upper direction by a coil spring wound around the nozzle body, the pushing body pushing the stem according to pushing down operation to open a fixed amount injection valve disposed in the aerosol container thereby allowing injection of entire amounts of aerosol contents within the fixed amount injection valve, the pushing body being pivotally movable with respect to the nozzle body and the outer sleeve; and
an upper sleeve attaching the pushing body slidably in an up and down direction at a center opening thereof, the upper sleeve being secured to the outer sleeve at a lower end thereof,
wherein plural lower receiving blades whose top end forms a portion tapered at one corner are arranged annularly at an outer periphery of the penetration opening of the outer sleeve via lower insertion intervals extending in an up and down direction, wherein a flat portion having the same level as the lower receiving blades is formed at an end of the lower receiving blades in this arrangement direction, wherein a fitting piece is formed in projecting from a lower surface of the pushing body for allowing the pushing body to be pushed to the stem by pushing the pushing body to the lower receiving blades from an upper surface of the blades along the portion tapered at one corner as to be moved and inserted into the lower insertion interval, wherein plural upper receiving blades for moving the pushing body in the same direction as the moving direction of the pushing body by hitting a top of the fitting piece according to the pushing body's lifting up due to release of pushing operation to the pushing body are arranged annularly at an inner surface of the upper sleeve in having a lower surface tapered at one corner via upper insertion intervals, and wherein the fixed amount injection of the aerosol contents is disabled by pushing operation plural times to the pushing body and by disabling movement of the pushing body into the lower insertion interval upon rendering the fitting piece hit the flat portion after the pushing body is moved according to the pushing operation.

2. The subdivided fixed amount distributing apparatus for aerosol container according to claim 1, wherein the pushing body is formed with a pushing projection at an upper surface thereof to be in pressurized contact with a user.

3. The subdivided fixed amount distributing apparatus for aerosol container according to claim 2, wherein the pushing projection of the pushing body is formed in coupling with the fitting piece.

4. The subdivided fixed amount distributing apparatus for aerosol container according to claim 2, wherein the pushing body is formed with the pushing projection and the fitting piece, which are formed separately.
